# EUROPÄISCHE PATENTSCHRIFT

(11) **EP 1 161 406 B1**
(45) Veröffentlichungstag und Bekanntmachung des Hinweises auf die Patenterteilung: **21.04.2004**
(21) Anmeldenummer: 99952553.8
(22) Anmeldetag: 12.10.1999
(51) Int. Cl.: C07C 17/02

(54) **VERFAHREN ZUR HERSTELLUNG VON ETHYLEN(DI)CHLORID (EDC)**
METHOD OF PRODUCING ETHYLENE (DI)CHLORIDE (EDC)
PROCEDE POUR PRODUIRE DU (DI)CHLORURE D'ETHYLENE (EDC)

(30) Priorität: 12.03.1999 DE 19910964
(43) Veröffentlichungstag der Anmeldung: 12.12.2001
(73) Patentinhaber: Uhde GmbH, 44141 Dortmund (DE); Vinnolit Technologie GmbH & Co. KG, 84504 Burgkirchen (DE)
(72) Erfinder: BENJE, Michael, D-64289 Darmstadt (DE)
(74) Vertreter: Dabringhaus, Walter, Dipl.-Ing.
(86) Internationale Anmeldenummer: PCT/EP1999/007649
(87) Internationale Veröffentlichungsnummer: WO 2000/055107

(56) Entgegenhaltungen:
- EP-A- 0 026 349
- EP-A- 0 471 987
- DE-A- 2 427 045
- US-A- 3 941 568
- US-A- 4 554 392
- DATABASE WPI Section Ch, Week 199608 Derwent Publications Ltd., London, GB; Class E16, AN 1996-074782 XP002128146 & JP 07 330639 A (TOSOH CORP), 19. Dezember 1995 (1995-12-19)
- PATENT ABSTRACTS OF JAPAN vol. 018, no. 482 (C-1247), 8. September 1994 (1994-09-08) & JP 06 157365 A (TOSOH CORP), 3. Juni 1994 (1994-06-03)

## Beschreibung

Die Erfindung richtet sich auf ein Verfahren zur Herstellung von 1,2-Dichlorethan bzw. Ethylen(di)chlorid (EDC) unter Einsatz eines im Umlauf geführten Reaktionsmediums sowie eines Katalysators, wobei dem Reaktionsmedium Ethylen und Chlor zugeführt werden.

Die großtechnische Herstellung von 1,2-Dichlorethan geschieht durch Einbringen der gasförmigen Reaktionspartner Chlor und Ethylen in ein umlaufendes flüssiges Reaktionsmedium (meist 1,2-Dichlorethan), das einen geeigneten Katalysator (z.B. Eisen(III)-chlorid) in gelöster Form enthält. Bei der Ausführung der hierzu verwendeten Reaktionssysteme kann man unterscheiden zwischen Konzepten, bei denen das Reaktionsmedium umgepumpt wird und solchen, bei denen das Reaktionsmedium sowohl durch den Mammutpumpeneffekt der eindosierten, gasförmigen Reaktionspartner als auch durch den durch die Reaktionswärme erzeugten Naturumlauf umgewälzt wird.

Ein System der ersten Art wird z.B. in der DE-19 05 517, DE-25 42 057 und der DE-40 39 960-A1 beschrieben. Das Reaktionsmedium wird hier über einen äußeren Kreislauf umgepumpt und saugt mittels eines Flüssigkeitsstrahl-Gasverdichters zunächst das gasförmige Chlor an. Danach wird gasförmiges Ethylen durch einen gelochten Gasverteiler eingespeist. Der so erzeugte Mischstrom durchströmt nun eine Füllkörperpackung oder einen statischen Mischer, an dem die durch den Gasverteiler erzeugten, relativ großen Ethylenblasen dispergiert werden, so daß das Ethylen sich mit hinreichender Geschwindigkeit löst und mit dem bereits gelösten Chlor reagiert.

Die Verwendung eines statischen Mischers wird in EP-0 471 987-B1 beschrieben. Die Füllkörperpackung bzw. der statische Mischer stellen somit die eigentliche Reaktionsstrecke dar.

Neben dem Ansaugen und Feinverteilen bzw. dem Lösen der Reaktionspartner erfüllt der umgepumpte Strom noch eine weitere Aufgabe: die Abfuhr der Reaktionswärme. Die Reaktionswärme beträgt etwa 2.200 kJ/kg EDC, d.h. bei der Produktion von 1 t EDC fällt eine Wärmemenge an, die ausreicht, um ca. 1 t Wasserdampf zu erzeugen. Daher muß die umgewälzte Menge groß genug sein, daß nicht längs der Reaktionsstrecke Sieden eintritt.

Bei dem in den oben zitierten Schriften beschriebenen System siedet der Reaktorinhalt nicht. Der heiße Umlaufstrom wird vielmehr in zwei Teilströme aufgeteilt. Der größere Teilstrom dient zur indirekten Beheizung von Kolonnen, während der kleinere Teilstrom teilweise entspannt wird. Der durch die Entspannungsverdampfung erzeugte, dampfförmige Strom entspricht der produzierten Menge und kann zur Aufreinigung direkt in eine Kolonne eingespeist werden.

Ein besonderer Nachteil der bekannten Verfahrensweise besteht darin, daß der Umpumpstrom sehr groß ist und große und leistungsstarke Pumpen erforderlich sind, die hohe Investitions- und Betriebskosten bedingen.

Bei dem anderen System mit Naturumlauf wird der erforderliche Flüssigkeitsumlauf durch den Mammutpumpeneffekt der gasförmigen Reaktionspartner bzw. durch natürliche Konvektion infolge der freigesetzten Reaktionswärme erzeugt. Der Flüssigkeitsumlauf kann dabei über eine äußere Leitung oder auch im Reaktionsgefäß selbst geschehen.

Dieses Verfahren ist z.B. in der DE-OS 24 27 045 beschrieben. Hier läuft das Reaktionsmedium über eine äußere Leitung um und die Reaktionspartner werden über Gasverteiler in den Kreislaufstrom eingebracht. Danach durchströmt das Reaktionsgemisch eine Packung, die die Reaktionsstrecke darstellt. Die Umlaufmenge kann durch eine Armatur gesteuert werden. Bei diesem Konzept siedet der Reaktorinhalt. Daher muß durch eine ausreichende geodätische Höhe, eine entsprechende Positionierung der Reaktionsstrecke und auch durch Einstellung eines ausreichenden Umlaufstromes sichergestellt werden, daß längs der Reaktionsstrecke kein Sieden auftritt. Ein Nachteil dieses Systems ist, daß das Chlor nicht durch einen Flüssigkeitsstrahl-Gasverdichter angesaugt und verdichtet wird, sondern schon mit einem gewissen Mindestdruck zur Verfügung stehen muß, was oftmals nicht der Fall ist und eine Vorverdichtung des Chlors erforderlich machen kann.

Beiden Systemen ist zu eigen, daß am Beginn der Reaktionsstrecke ein lokaler Chlorüberschuß vorliegen kann, der die Bildung von höher chlorierten Nebenprodukten begünstigt. Dies hat seine Ursache darin, daß Chlor in 1,2-Dichlorethan um ein mehrfaches besser löslich ist als Ethylen. Bei den beiden beschriebenen Systemen hat dies folgende Konsequenzen:

Beim ersten System (Zwangsumlauf durch Pumpen) wird das Chlor durch die hohen, im Flüssigkeitsstrahl-Gasverdichter wirkenden Scherkräfte sofort sehr fein verteilt und erreicht die Zugabestelle des Ethylens schon weitgehend gelöst. Am Beginn der Ethyleneinlösung liegt also schon ein Chlorüberschuß vor.

Beim zweiten System (Naturumlauf) löst sich das zugegebene Chlor längs der Reaktionsstrecke schneller auf als das Ethylen. Auch hier besteht zumindet am Beginn der Reaktionsstrecke ein Chlorüberschuß.

EP-A-26349 beschreibt ein Verfahren zur Herstellung von EDC unter Einsatz eines im Umlauf geführten Reaktionsmediums sowie eines Katalysators.

Aufgabe der Erfindung ist die Schaffung einer Lösung, mit der die katalytische Chlorierung von Ethylen in besonders produktschonender Weise ermöglicht wird, wobei die bei der Reaktion entstehende Reaktionswärme nutzbringend eingesetzt und die unerwünschte Bildung höher chlorierter Produkte, wie Tri-, Tetra- und Pentachlorethan im Reaktor weitgehend vermieden wird bei einer anlagemäßigen Gestaltung, die eine modulare, kostengünstige Systemerweiterung zuläßt.

Mit einem Verfahren der eingangs bezeichneten Art wird diese Aufgabe gemäß der Erfindung dadurch gelöst, daß in Umlaufrichtung des Reaktionsmediums gesehen an einer stromaufwärtigen Stelle Ethylen derart in das umlaufende Medium eingeleitet wird, daß es nach Durchlaufen einer Misch- und Lösezone in dem Reaktionsmediumstrom vollständig aufgelöst ist, und daß das Chlor in einem gekühlten Teilstrom des Reaktionsmediums aufgelöst und dann dem Hauptstrom des Reaktionsmediums weiter stromabwärts zugeführt wird, wobei das mit Hilfe der bei der Reaktion von Chlor und Ethylen freiwerdende, mit der Reaktionswärme verdampfte Ethylen(di)-chlorid aus dem Reaktionsgefäß dampfförmig abgeführt wird, während der im Ausdampfgefäß verbleibende Rest im Umlauf zur Reaktionszone zurückgeführt wird.

Vorzugsweise wird das Ethylen oder Chlorgas mittels mikroporöser Gasverteilelemente zur Erzeugung von Gasblasen von 0,3 bis 3 mm Durchmesser in das Reaktionsmedium eingebracht.

Durch diese besondere Einbringungsart der Gase in das Reaktionsmedium wird eine ausreichende Feinverteilung erreicht und damit eine optimale Produktion. Das Einbringen dieser Gasblasen in der erfindungsgemäßen Größe ist vergleichsweise einfach möglich, wobei besondere Einperlvorrichtungen weiter unten näher angegeben sind.

Ein Beitrag zur Lösung der Aufgabe besteht auch darin, daß das Chlor in einem gekühlten Teilstrom des Reaktionsmediums aufgelöst wird und dann dem Hauptstrom des Reaktionsmediums zugeführt wird. Durch diese Maßnahme läßt sich ebenfalls die erfindungsgemäße Aufgabe lösen, nämlich die katalytische Chlorierung von Ethylen in besonders produktschonender Weise.

Schließlich gehört zur Erfindung auch, daß das mit Hilfe der bei der Reaktion von Chlor und Ethylen freiwerdende, mit der Reaktionswärme verdampfte Ethylen(di)chlorid aus dem Reaktionsgefäß dampfförmig abgeführt wird, während der im Ausdampfgefäß verbleibende Rest im Umlauf zur Reaktionszone zurückgeführt wird.

Mit dem erfindungsgemäßen Verfahren lassen sich optimale Ergebnisse erreichen, da die am Verfahren beteiligten Partner jeweils ausreichend Zeit haben zu reagieren. So kann beispielsweise das Ethylen zwischen Ethylenverteiler einerseits und dem Beginn des Chlorverteilers andererseits die freie Lösungsstrecke durchlaufen und sich dabei durch die kleine Anfangsblasengröße, erzeugt durch die mikroporösen Gasverteilelemente, vollständig auflösen, so daß die nachfolgende Reaktion in Lösung stattfindet.

Natürlich kann neben einer Ethylen-Zugabestelle auch mehrere Zugabestellen vorgesehen sein. In jedem Falle verbleibt das gebildete EDC zunächst in der flüssigen Phase und verdunstet erst an oder im Bereich der Oberfläche des Ausdampfgefäßes, wobei die Verdunstungskälte durch die Reaktionswärme kompensiert wird.

Die Reaktionspartner Ethylen und Chlor können durch inerte Gase verdünnt sein. Als Katalysator kann sich beispielsweise die Verwendung von Eisen(III)-chlorid empfehlen. Zur Vermeidung einer Nebenproduktbildung kann als Inhibitor z.B. auch Sauerstoff eingesetzt werden.

Ausgestaltungen der erfindungsgemäßen Verfahrensweise ergeben sich aus den Unteransprüchen, wobei es zweckmäßig ist, z.B. als Reaktionsmedium ein überwiegend 1,2-Dichlorethan enthaltendes Medium einzusetzen. Als Verfahrensparameter bieten sich an, in der Misch- und Reaktionszone eine Temperatur von 75° bis 200°C und einen Druck von 1 bis 15 bar einzustellen und die Durchflußgeschwindigkeit so zu steuern, daß eine Verweilzeit des Reaktionsgemisches in der Misch- und Reaktionszone 1 bis 30 Sekunden beträgt.

Schließlich ist auch vorgesehen, daß am Ende der Misch- und Lösungszone das Chlor, welches zuvor in einem unterkühlten Teilstrom des Reaktionsmediums aufgelöst wurde, dem Hauptstrom des Reaktionsmediums zugeführt wird. Die Auflösung des Chlors in einem unterkühlten Teilstrom hat den Vorteil, daß sich dann besonders viel Chlor löst und man mit einer vergleichsweise geringen Flüssigkeitsmenge bzw. kleineren Pumpen auskommen kann. Hierbei wird die steigende Löslichkeit des Chlors mit sinkender Temperatur vorteilhaft genutzt.

Alternativ kann aber auch vorgesehen sein, daß das Chlor in einem getrennten Flüssigkeitskreislauf in z.B. 1,2-Dichlorethan gelöst und dem Reaktionsmedium zugegeben wird.

Zur Lösung der Aufgabe sieht die Erfindung auch eine Anlage zur Durchführung des erfindungsgemäßen Verfahrens vor, die sich auszeichnet durch einen Ausdampfbehälter, ein Fallrohr, das über eine Übergangsleitung in ein Steigrohr übergeht, wobei im Steigrohr in Strömungsrichtung zunächst eine Ethyleneinspeisung, nachfolgend eine Auflösungszone und daran anschließend Verteilerrohre sind, und einen Bypass für das Reaktionsmedium aus dem Ausdampfbehälter, wobei dieser Bypass mit einer Pumpe, einem der Abkühlung dieses Teilstromes dienenden Wärmetauscher, einem nachfolgenden Flüssigkeitsstrahlverdichter zur Ansaugung und Einbringung von gasförmigem oder flüssigen Chlor in den Bypass-Strom sowie einer Zuführung in eine Ringleitung mit Verteilerrohren zum Einbringen des Bypass-Stromes in den Hauptstrom ausgestattet ist.

Weitere Ausgestaltungen ergeben sich aus den weiteren Unteransprüchen zur erfindungsgemäßen Anlage. So kann vorgesehen sein, daß im Strömungsweg des Reaktionsmediums zur Erzeugung eines Zwangsumlaufes eine Umwälzeinrichtung und zur Steuerung eine Drosselklappe od. dgl. vorgesehen ist. An dieser Stelle sei bemerkt, daß der Näturumlauf, wobei in dessen Strömungsweg ähnliche Steuerelemente vorgesehen sein können, mit Gegenstand der vorliegenden Erfindung ist.

Um eine sehr präzise Steuerung des Durchflusses vornehmen zu können, sieht die Erfindung in Ausgestaltung auch vor, daß zur Messung des Durchflusses im Hauptstrom eine Ultraschall-Meßeinrichtung vorgesehen ist sowie eine Steuerung zur Betätigung einer Durchflußregelklappe od. dgl.

Einem Fallrohr können wenigstens zwei Steigrohre mit den erfindungsgemäßen Einbauten zugeordnet sein. Auch kann eine Mehrzahl von Ausdampfgefäßen mit einem oder mehreren Fallund Steigrohren erfindungsgemäß in der entsprechenden Art und Weise angeordnet sein, wobei dort eine oder mehrere Reaktionszonen in der oder den Umlaufleitungen angeordnet sind.

Diese Ausgestaltungen machen es möglich, die Anlage in einer Art Modulbauweise zu gestalten. Hierzu ist es vorteilhaft, wenn jede Einheit aus Ausdampfgefäß, Fallrohr und Steigrohr mit Einbauten als Modul ausgebildet ist mit Einrichtungen zur Kopplung wenigstens eines Nachbarmoduls oder deren mehrerer. Eine andere bevorzugte Ausgestaltung ist die eines Ausdampfgefäßes mit mehreren Steig- und Fallrohren oder mit einem zentralen Fallrohr und mehreren, von diesem ausgehenden Steigrohren.

Weitere Ausgestaltungen der Erfindung bestehen darin, daß im Bypass ein Mischer mit Wärmetauscher als vorrichtungsmäßige Einheit vorgesehen ist und/oder daß im Hauptstrom mikroporöse Begasungselemente zur Feinverteilung des einzubringenden Ethylens vorgesehen sind und/oder daß in der Reaktionszone strömungsgleichrichtende Einbauten, wie Leitbleche, Drosselklappen od. dgl., vorgesehen sind.

Eine weitere vorteilhafte Gestaltung von Elementen der erfindungsgemäßen Anlage besteht darin, daß vor den Begasungselementen ein Strömungsgleichrichter zur Vergleichmäßigung eines Geschwindigkeitsprofiles sowie zur Unterdrükkung radialer Geschwindigkeitskomponenten im Hauptstrom angeordnet ist.

Weitere Merkmale, Einzelheiten und Vorteile der Erfindung ergeben sich aufgrund der nachfolgenden Beschreibung sowie anhand der Zeichnungen. Diese zeigen in
- Fig. 1: ein vereinfachtes Anlagenschaltbild nach der Erfindung,
- Fig. 2: einen teilweise vergrößerten Ausschnitt aus einem Steigrohr der erfindungsgemäßen Anlage mit symbolisch angedeuteten Einbauten,
- Fig. 3 und 4: vereinfachte Schnittzeichnungen gemäß Linien III-III bzw. IV-IV in Fig. 2,
- Fig. 5: eine Teilansicht eines Rohrbereiches mit einer Treibstrahldüse sowie in
- Fig. 6: einen Teilrohrausschnitt mit einer Stickstoffeindüsung.

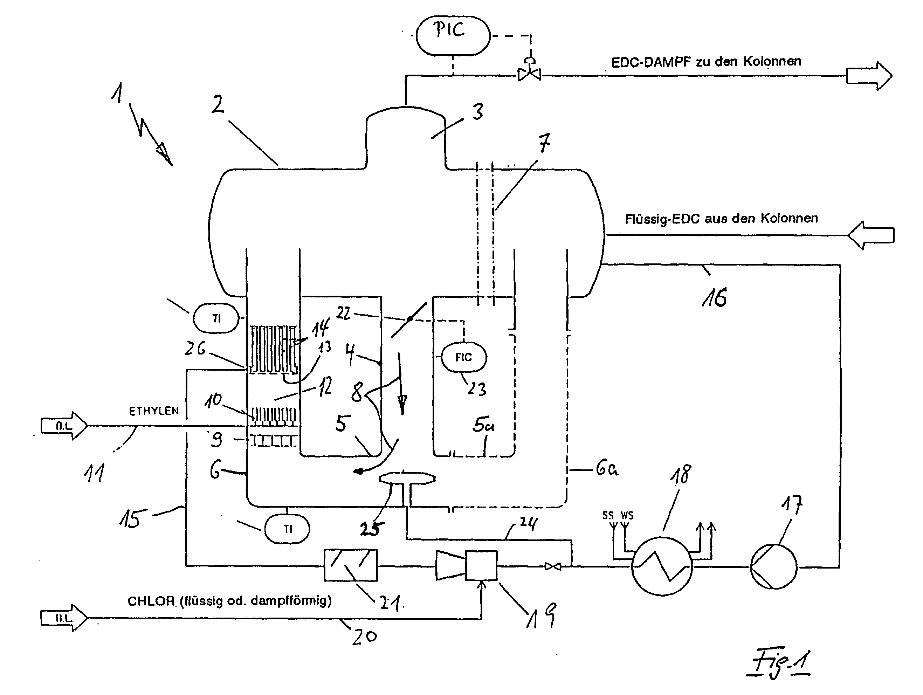

Die allgemein mit 1 bezeichnete Anlage zur Durchführung des Verfahrens zur Herstellung von EDC unter Einsatz eines im Umlauf geführten Reaktionsmediums ist in Fig. 1 im wesentlichen symbolhaft und stark vereinfacht wiedergegeben.

So zeigt die Fig. 1 einen Ausdampfbehälter 2 mit einem angedeuteten Dampfdom 3 und einem Fallrohr 4, das über eine Übergangsleitung 5 in ein allgemein mit 6 bezeichnetes Steigrohr übergeht, das wiederum im Ausdampfbehälter 2 mündet. Strichpunktiert ist in der Figur, dem Fallrohr nach rechts folgend, eine Übergangsleitung 5a und ein Steigrohr 6a zugeordnet, wobei der Ausdampfbehälter 2 über strichpunktierte Linien 7 geteilt ist, womit angedeutet ist, daß die Anlage in Modulbauweise gestaltet sein kann, d.h. es können mehrere Steigrohre an einem modulhaft verlängerten Ausdampfbehälter 2 vorgesehen sein, auch mit ggf. mehr als einem Fallrohr, worauf es hier nicht näher ankommt.

Wie sich aus Fig. 1 ergibt, folgt die Strömung entweder durch Natur- oder Zwangsumlauf einer bestimmten Richtung, die dort mit den Pfeilen 8 angegeben ist. Nach Durchlaufen der Übergangsleitung 5 ist im Steigrohr 6 in Strömungsrichtung zunächst ein Strömungsgleichrichter 9 vorgesehen, um das axiale Geschwindigkeitsprofil zu vergleichmäßigen und radiale Geschwindigkeitskomponenten zu unterdrücken. Diesem Strömungsgleichrichter 9 folgt eine Reihe von mikroporösen Begasungselementen 10, über die Ethylen über die Leitung 11 in das umlaufende Reaktionsmedium eingeperlt werden kann.

Anschließend ist im Steigrohr eine Lösezone, mit 12 bezeichnet, vorgesehen, der wiederum ein Strömungsvergleichsmäßigungseinbau 13 und ein Einspeiselement 14 für im Reaktionsmedium gelöstes Chlor nachgeordnet sind. Die Zuführleitung dieses Reaktionsmedium/Chlor-Gemisches ist mit 15 bezeichnet.

Über eine Leitung 16 wird ein Teilstrom als Bypass-Strom 16 dem Reaktionsmedium entnommen, von einer Pumpe 17 einem Kühler 18 zugeführt, wobei über einen Flüssigkeitsstrahlverdichter 19 das über die Leitung 20 zugeführte Chlor diesem Bypass-Strom beigemischt wird, wobei im Strömungsweg noch ein statischer Mischer 21 vorgesehen sein kann.

Zur Regelung des Umlaufes ist im Fallrohr 4 eine symbolisch angeordnete Drosselklappe 22 vorgesehen, deren Stellung beispielsweise über eine Ultraschall-Meßmethode des Durchflusses, allgemein mit 23 bezeichnet, erfolgt.

Zum Anfahrbetrieb kann ein Teilstrom des Bypass-Stromes 16 über die Leitung 24 einer Verteilerdüse 25 zugeführt werden, die im Übergangsbereich 5 vom Fallrohr 4 auf das Steigrohr 6 für die notwendige Strömung sorgt, wobei der Wärmetauscher 18 dann auch als Heizer eingesetzt werden kann.

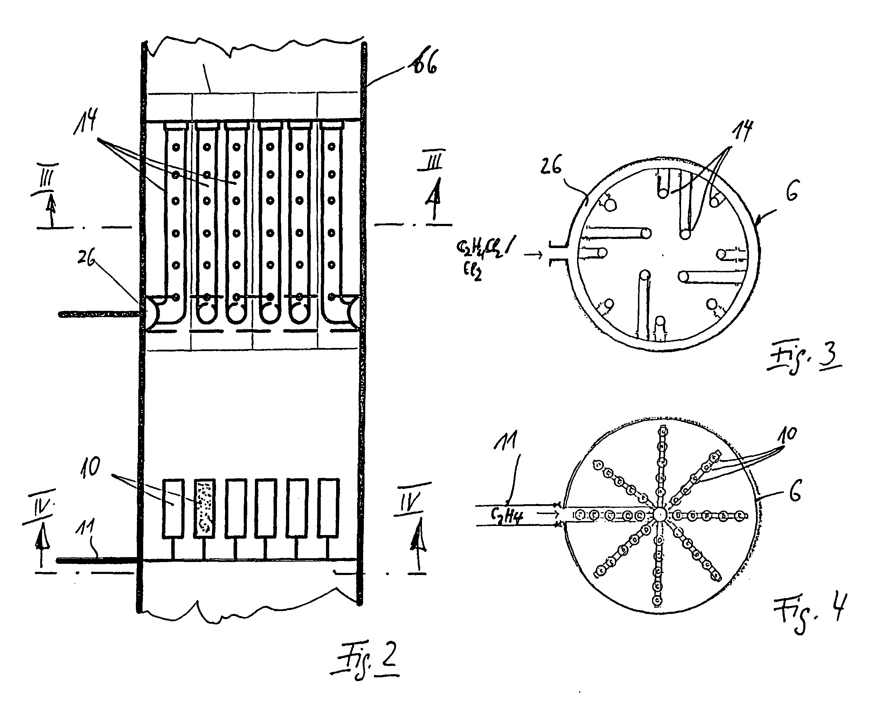

Die Anordnung und Positionierung der Begasungselemente 10 sowie der Einspeiselemente 14 für das Ethylen einerseits und das Reaktionsmedium/Chlor-Gemisch andererseits sind in den Fig. 2 bis 4 etwas näher dargestellt, wobei dort nur Beispiele wiedergegeben sind.

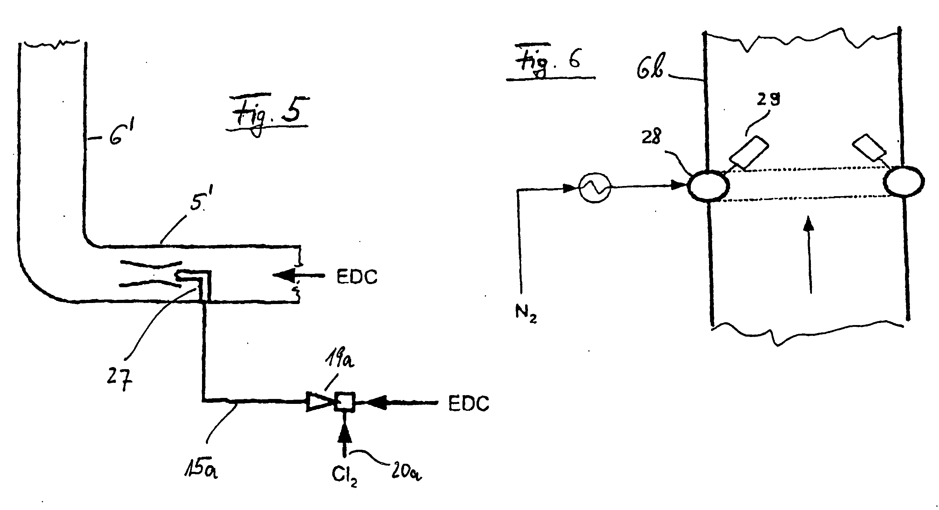

Die mikroporösen Begasungselemente 10 werden, wie sich aus Fig. 2 in Verbindung mit Fig. 4 ergibt, von der Leitung 11 beaufschlagt, sind sternförmig im Inneren des Steigrohres 6 angeordnet, wobei diese Anordnung nicht zwingend ist. Demgegenüber sind die Einspeiselemente 14 an einem Ringkanal 26 angebracht, auch dies ist in Fig. 2 in Verbindung mit Fig. 3 lediglich angedeutet.

In Fig. 5 ist die Möglichkeit dargestellt, in der Übergangsleitung, dort mit 5' bezeichnet, eine Düse 27 vorzusehen, über die derjenige EDC-Teilstrom 15' eingedüst wird, in den zuvor das Chlor 20' z.B. über einen Flüssigkeitsstrahlverdichter 19' eingebracht wurde. Dieser Teilstrom kann als Treibstrahl benutzt werden, um die Strömung im Schlaufenreaktor 1 zu unterstützen.

Fig. 6 zeigt die Möglichkeit, zur Verhinderung von Siedeverzügen (Schwallströmung) kleine Mengen an vorgewärmtem Stickstoff über eine Ringleitung 28 z.B. am Steigrohr 6b vorzusehen, wobei die Ringleitung 28 mit Kerzen 29 aus einem rauhen porösen Material versehen ist, wie dies in Fig. 6 angedeutet ist.

Natürlich ist das beschriebene Ausführungsbeispiel der Erfindung noch in vielfacher Hinsicht abzuändern, ohne den Grundgedanken zu verlassen. So ist die Erfindung insbesondere nicht auf die hier dargestellte spezielle Anordnung und Gestaltung der Begasungselemente 10 einerseits und der Einspeiselemente 14 andererseits beschränkt, auch nicht auf eine bestimmte Art der Steuerung, wobei die hier gewählte allerdings besonders zweckmäßig ist. Die Begasungselemente können auch im Fallrohr vorgesehen sein, da die Ethylenauflösung auch im Fallrohr stattfinden kann, wobei die erzeugten Gasblasen durch den Flüssigkeitsstrom abwärts transportiert werden. (Die durch den Umlauf erzeugte Strömungsgeschwindigkeit der Flüssigkeit ist größer als die Aufstiegsgeschwindigkeit der Gasblasen).

## Patentansprüche

1. Verfahren zur Herstellung von 1,2-Dichlorethan bzw. Ethylen(di)chlorid (EDC) unter Einsatz eines im Umlauf geführten Reaktionsmediums sowie eines Katalysators, wobei dem Reaktionsmedium Ethylen und Chlor zugeführt werden,
**dadurch gekennzeichnet,**
- **daß** in Umlaufrichtung des Reaktionsmediums gesehen an einer stromaufwärtigen Stelle Ethylen derart in das umlaufende Medium eingeleitet wird, daß es nach Durchlaufen einer Misch- und Lösezone in dem Reaktionsmediumstrom vollständig aufgelöst ist, und
- **daß** das Chlor in einem gekühlten Teilstrom des Reaktionsmediums aufgelöst und dann dem Hauptstrom des Reaktionsmediums weiter stromabwärts zugeführt wird,
- wobei das mit Hilfe der Reaktion von Chlor und Ethylen freiwerdende, mit der Reaktionswärme verdampfte Ethylen(di)chlorid aus dem Reaktionsgefäß dampfförmig abgeführt wird, während der im Ausdampfgefäß verbleibende Rest im Umlauf zur Reaktionszone zurückgeführt wird.

2. Verfahren nach Anspruch 1,
**dadurch gekennzeichnet,**
**daß** als Reaktionsmedium ein überwiegend 1,2-Dichlorethan enthaltendes Medium eingesetzt wird.

3. Verfahren nach Anspruch 1 oder 2,
**dadurch gekennzeichnet,**
**daß** in der Misch- und Reaktionszone eine Temperatur von 75° bis 200°C und ein Druck von 1 bis 15 bar eingestellt und die Durchflußgeschwindigkeit so gesteuert wird, daß eine Verweilzeit des Reaktionsgemisches in der Misch- und Reaktionszone von 1 bis 30 Sekunden gegeben ist.

4. Verfahren nach einem der vorangehenden Ansprüche,
**dadurch gekennzeichnet,**
**daß** die Einbringung des Ethylen oder Chlorgases mittels mikroporöser Gasverteilelemente zur Erzeugung von Gasblasen von 0,3 bis 3 mm Durchmesser in das Reaktionsmedium vorgenommen wird.

5. Verfahren nach einem der vorangehenden Ansprüche,
**dadurch gekennzeichnet,**
**daß** das gebildete EDC zunächst in der flüssigen Phase verbleibt und erst an oder im Bereich der Oberfläche des Ausdampfgefäßes verdunstet, wobei die Verdunstungskälte durch die Reaktionswärme kompensiert wird.

6. Verfahren nach einem der vorangehenden Ansprüche,
**dadurch gekennzeichnet,**
**daß** das Chlor getrennt in der Flüssigkeit gelöst und dem Reaktionsmedium zugegeben wird.

7. Anlage zur Durchführung des Verfahrens nach einem der vorangehenden Ansprüche,
**gekennzeichnet durch**
einen Ausdampfbehälter (2), ein Fallrohr (4), das über eine Übergangsleitung (5) in ein Steigrohr (6) übergeht, wobei im Steigrohr (6) in Strömungsrichtung zunächst eine Ethyleneinspeisung (10), nachfolgend eine Auflösungszone (12) und daran anschließend Verteilerrohre (14) sind, und einen Bypass (16) für das Reaktionsmedium aus dem Ausdampfbehälter (2), wobei dieser Bypass (14) mit einer Pumpe (17), einem der Abkühlung dieses Teilstromes dienenden Wärmetauscher (18), einem nachfolgenden Flüssigkeitestrahlverdichter (19) zur Ansaugung und Einbringung von gasförmigem oder flüssigen Chlor in den Bypass-Strom sowie einer Zuführung in eine Ringleitung (26) mit Verteilerrohren (14) zum Einbringen des Bypass-Stromes in den Hauptstrom ausgestattet ist.

8. Anlage nach Anspruch 7,
**dadurch gekennzeichnet,**
**daß** im Strömungsweg des Reaktionsmediums zur Erzeugung eines Zwangsumlaufes eine Umwälzeinrichtung und zur Steuerung eine Drosselklappe (22) od. dgl. vorgesehen ist.

9. Anlage nach Anspruch 7 oder einem der folgenden,
**dadurch gekennzeichnet,**
**daß** zur Messung des Durchflusses im Hauptstrom eine Ultraschall-Meßeinrichtung (23) vorgesehen ist sowie eine Steuerung zur Betätigung einer Durchflußregelklappe (22) od. dgl.

10. Anlage nach Anspruch 7 oder einem der folgenden,
**dadurch gekennzeichnet,**
**daß** einem Fallrohr (4) wenigstens zwei Steigrohre (6,6a) mit den erfindungsgemäßen Einbauten zugeordnet ist.

11. Anlage nach Anspruch 7 oder einem der folgenden,
**gekennzeichnet durch**
eine Mehrzahl von Ausdampfgefäßen (2) mit einem oder mehreren Fall- und Steigrohren (4,6), wobei dort eine oder mehrere Reaktionszonen (12) in der oder den Umlaufleitungen angeordnet sind.

12. Anlage nach Anspruch 7 oder einem der folgenden,
**dadurch gekennzeichnet,**
**daß** jede Einheit aus Ausdampfgefäß (2), Fallrohr (4) und Steigrohr (6) mit Einbauten als Modul ausgebildet ist mit Einrichtungen zur Kopplung wenigstens eines Nachbarmoduls oder mehrerer ausgebildet sind.

13. Anlage nach Anspruch 7 oder einem der folgenden,
**dadurch gekennzeichnet,**
**daß** im Bypass (16) ein Mischer mit Wärmetauscher als vorrichtungsmäßige Einheit vorgesehen ist.

14. Anlage nach Anspruch 7 oder einem der folgenden,
**dadurch gekennzeichnet,**
**daß** im Hauptstrom mikroporöse Begasungselemente (10) zur Feinverteilung des einzubringenden Ethylens vorgesehen sind.

15. Anlage nach Anspruch 7 oder einem der folgenden,
**dadurch gekennzeichnet,**
**daß** in der Reaktionszone (12) strömungsgleichrichtende Einbauten, wie Leitbleche (13), Drosselklappen od. dgl., vorgesehen sind.

16. Anlage nach Anspruch 7 oder einem der folgenden,
**dadurch gekennzeichnet,**
**daß** vor den Begasungselementen (10) ein Strömungsgleichrichter (9) zur Vergleichmäßigung eines Geschwindigkeitsprofiles sowie zur Unterdrückung radialer Geschwindigkeitskomponenten im Hauptstrom angeordnet ist.

17. Anlage nach Anspruch 7 oder einem der folgenden,
**dadurch gekennzeichnet,**
**daß** zur Einbringung der konzentrierten Chlorlösung in den Hauptumlaufstrom eine Düse im Schlaufenreaktor angeordnet ist.

18. Anlage nach Anspruch 7 oder einem der folgenden,
**dadurch gekennzeichnet,**
**daß** zur Einbringung kleiner Mengen an vorgewärmtem Stickstoff an einem Verteilerring (28) im Steigrohr (6b) des Schlaufenreaktors Kerzen (29) aus einem rauhen, porösen Material, vorzugsweise Keramik oder Sintermetall, vorgesehen sind.

## Claims

1. Process for producing 1,2-dichloroethane or ethylene (di)chloride (EDC) using a circulating reaction medium and a catalyst, with ethylene and chlorine being supplied to the reaction medium,
**characterised in that**
- at an upstream location as viewed in the direction of circulation of the reaction medium, ethylene is passed into the circulating medium in such a manner that after passing through a mixing and dissolving zone it is completely dissolved in the stream of reaction medium, and
- the chlorine is dissolved in a cooled component stream of the reaction medium and then supplied to the principal stream of the reaction medium at a point further downstream,
- the ethylene (di)chloride being liberated with the aid of the reaction of the chorine and ethylene, and evaporated by the heat of reaction, is withdrawn as vapour from the reaction vessel, whereas the residue remaining in the evaporation vessel is recirculated back to the reaction zone.

2. Process according to claim 1,
**characterised in that**
a medium containing predominantly 1,2-dichloroethane is employed as the reaction medium.

3. Process according to claim 1 or 2,
**characterised in that**
a temperature of 75° to 200° C and a pressure of 1 to 15 bars are adjusted in the mixing and reaction zone and the throughput rate is controlled in such a way that the reaction mixture enjoys a residence time of 1 to 30 seconds in the mixing and reaction zone.

4. Process according to any of the preceding claims,
**characterised in that**
the ethylene or chlorine gas is introduced into the reaction medium by means of microporous gas distributing elements so as to generate gas bubbles with a diameter of 0.3 to 3 mm.

5. Process according to any of the preceding claims,
**characterised in that**
the EDC formed remains initially in liquid phase and only vaporises at or in the region of the surface of the evaporation vessel, with the cold due to evaporation being compensated by the heat of reaction.

6. Process according to any of the preceding claims,
**characterised in that**
the chlorine is separately dissolved in the liquid and added to the reaction medium.

7. Installation for carrying out the process according to any of the preceding claims,
**characterised by**
an evaporation tank (2), a downpipe (4) which merges into a riser (6) via an adapter line (5), the riser (6) featuring, in the direction of flow, first of all an ethylene feed (10), then a dissolution zone (12), followed by distribution pipes (14), and by a bypass (16) for the reaction medium from the evaporation tank (2), said bypass (14) being equipped with a pump (17), a heat exchanger (18) which is used to cool said component stream, thereafter a fluid stream compressor (19) for aspirating and introducing gaseous or liquid chlorine into the bypass stream, and also a feed into a ring line (26) with distribution pipes (14) for introducing the bypass stream into the principal stream.

8. Installation according to claim 7,
**characterised in that**
provided in the flow path of the reaction medium are a recirculating device for generating a forced circulation and a butterfly valve (22) or the like for control purposes.

9. Installation according to claim 7 or a following claim,
**characterised in that**
to meter the throughflow an ultrasonic metering device (23) is provided in the principal stream, along with a controller for operating a throughflow regulating valve (22) or the like.

10. Installation according to claim 7 or a following claim,
**characterised in that**
operatively associated with a downpipe (4) are at least two risers (6, 6a) featuring the baffles according to the invention.

11. Installation according to claim 7 or a following claim,
**characterised by**
a plurality of evaporation vessels (2) having one or more downpipes and risers (4, 6), one or more reaction zones (12) being disposed thereat in the circulator line or lines.

12. Installation according to claim 7 or a following claim,
**characterised in that**
each unit comprising an evaporation vessel (2), downpipe (4) and riser (6) is constructed with baffles as a module, and features devices for coupling at least one neighbouring module or more than one thereof.

13. Installation according to claim 7 or a following claim,
**characterised in that**
provided in the bypass (16) is a mixer with a heat exchanger constituting a unit of apparatus.

14. Installation according to claim 7 or a following claim,
**characterised in that**
provided in the principal stream are microporous gas delivery elements (10) for finely distributing the ethylene that is being introduced.

15. Installation according to claim 7 or a following claim,
**characterised in that**
provided in the reaction zone (12) are flow rectifying baffles such as deflector plates (13), butterfly valves or the like.

16. Installation according to claim 7 or a following claim,
**characterised in that**
disposed ahead of the gas delivery elements (10) is a flow rectifier (9) for evening out a velocity profile and for inhibiting radial velocity components in the principal stream.

17. Installation according to claim 7 or a following claim,
**characterised in that**
a nozzle is arranged in the loop reactor for introducing the concentrated chlorine solution into the principal circulating stream.

18. Installation according to claim 7 or a following claim,
**characterised in that**
plugs (29) of a rough, porous material, preferably a ceramic material or sintered metal, are provided on a distributor ring (28) in the riser (6b) of the loop reactor for introducing small quantities of pre-heated nitrogen.

## Revendications

1. Procédé de préparation de 1,2-dichloroéthane ou (di)chlorure d'éthylène (EDC) par utilisation d'un milieu de réaction circulant, ainsi qu'un catalyseur, où de l'éthylène et du chlore sont amenés dans le milieu de réaction, **caractérisé en ce que**
- en un site en amont vu dans la direction de la circulation du milieu de réaction, est introduit l'éthylène dans le milieu circulant, de sorte qu'il est complètement dissous dans le courant du milieu de réaction après passage d'une zone de mélange et de dissolution, et
- le chlore est dissous dans un courant partiel refroidi du milieu de réaction et est alors conduit dans le courant principal du milieu de réaction par la suite en amont,
- où le (di)chlorure d'éthylène, libéré par la réaction du chlore et de l'éthylène, évaporé avec la chaleur de réaction est évacué du récipient de réaction sous forme de vapeur, alors que la partie résiduelle restant dans le récipient de vaporisation est ramené dans la circulation vers la zone de réaction.

2. Procédé selon la revendication 1, **caractérisé en ce que** l'on met en oeuvre comme milieu de réaction, un milieu contenant essentiellement du 1,2-dichloroéthane.

3. Procédé selon la revendication 1 ou 2, **caractérisé en ce que** l'on règle dans la zone de mélange et de réaction, une température de 75° à 200°C et une pression de 1 à 15 bar et que la vitesse d'écoulement est réglée de sorte que l'on ait un temps de séjour du mélange réactionnel dans la zone de mélange et de réaction de 1 à 30 secondes.

4. Procédé selon l'une quelconque des revendications précédentes, **caractérisé en ce que** l'introduction de l'éthylène ou du chlore gazeux est réalisée dans le milieu de réaction à l'aide d'éléments microporeux de distribution des gaz pour produire des bulles de gaz de 0,3 à 3 mm de diamètre.

5. Procédé selon l'une quelconque des revendications précédentes, **caractérisé en ce que** le EDC formé reste d'abord en phase liquide et ne se volatilise que à côté ou dans la zone de la surface du récipient d'évaporation, où le froid de l'évaporation est compensé par la chaleur de réaction.

6. Procédé selon l'une quelconque des revendications précédentes, **caractérisé en ce que** le chlore est séparément dissous dans le liquide et ajouté au milieu de réaction.

7. Dispositif pour réaliser la mise en oeuvre du procédé selon l'une des revendications précédentes, **caractérisé par** un conteneur, un tuyau de descente (4), qui devient par une conduite de transition (5) un tuyau de montée (6), où dans le tuyau de montée (6) se trouvent, dans la direction du courant, d'abord une alimentation d'éthylène (10), ensuite une zone de dissolution (12) et puis, des tuyaux de distribution (14), et une dérivation (16) pour le milieu de réaction provenant du conteneur d'évaporation (2), où cette dérivation (16) est équipée d'une pompe (17), d'un échangeur de chaleur (18) servant au refroidissement de ce courant partiel, d'un condenseur de jet liquide (19) suivant pour l'admission et l'introduction de chlore gazeux ou liquide dans le courant de la dérivation, ainsi que d'une alimentation dans une conduite circulaire (26) avec tuyaux de distribution (14) pour l'introduction du courant de dérivation dans le courant principal.

8. Dispositif selon la revendication 7, **caractérisé en ce que** dans la voie du courant du milieu de réaction, une direction de circulation est prévue pour produire une circulation forcée, et une soupape d'étranglement (22) ou similaire pour le réglage.

9. Dispositif selon la revendication 7 ou l'une des suivantes, **caractérisé en ce que** pour la mesure du débit est prévu dans le courant principal un dispositif de mesure à ultrasons (23), ainsi qu'un réglage pour la mise en marche d'une soupape de réglage du débit (22) ou similaire.

10. Dispositif selon la revendication 7 ou l'une des suivantes, **caractérisé en ce qu'**à un tuyau de descente (4) est attribué à au moins deux tuyaux de montée (6, 6a) installés selon l'invention.

11. Dispositif selon la revendication 7 ou l'une des suivantes, **caractérisé par** une série de conteneurs d'évaporation (2) avec un ou plusieurs tuyaux de descente et de montée (4, 6), où une ou plusieurs zones de réaction (12) sont disposées dans la ou les conduites de circulation.

12. Dispositif selon la revendication 7 ou l'une des suivantes, **caractérisé en ce que** chaque unité comprenant un conteneur d'évaporation (2), un tuyau de descente (4) et un tuyau de montée (6) avec éléments est formée comme un module, avec arrangements pour le couplage à au moins un module adjacent ou plusieurs.

13. Dispositif selon la revendication 7 ou l'une des suivantes, **caractérisé en ce que** dans la dérivation (16), est prévu un mélangeur avec échangeur de chaleur comme unité de type dispositif.

14. Dispositif selon la revendication 7 ou l'une des suivantes, **caractérisé en ce que** dans le courant principal, sont prévus des éléments microporeux de volatilisation (10) pour distribuer finement l'éthylène introduit.

15. Dispositif selon la revendication 7 ou l'une des suivantes, **caractérisé en ce que** dans la zone de réaction (12), sont prévus des éléments dirigés dans le sens du courant, comme déflecteurs (13), soupapes d'étranglement, ou similaire.

16. Dispositif selon la revendication 7 ou l'une des suivantes, **caractérisé en ce que** avant les éléments de volatilisation (10), est disposé un redresseur de courant (9) pour l'homogénéisation d'un profil de vitesse ainsi que pour restreindre les composantes radiales de vitesse dans le courant principal.

17. Dispositif selon la revendication 7 ou l'une des suivantes, **caractérisé en ce que** pour l'introduction de la solution de chlore concentrée dans le courant principal de circulation, est disposée une buse dans le réacteur à boucle.

18. Dispositif selon la revendication 7 ou l'une des suivantes, **caractérisé en ce que** pour l'introduction de faibles quantités d'azote préchauffé sur un anneau de distribution (28) dans le tuyau de montée (6b) du réacteur à boucle, sont prévus des bougies (29) en un matériau rugueux, poreux, de préférence en céramique ou métal fritté.
